# EUROPEAN PATENT SPECIFICATION

(11) **EP 1 623 740 B1**
(45) Date of publication and mention of the grant of the patent: **07.05.2008**
(21) Application number: 04720576.0
(22) Date of filing: 15.03.2004
(51) Int. Cl.: A61P 13/08, A61K 36/889, A61K 131/00

(54) **PHARMACEUTICAL COMPOSITION AND METHOD FOR THE TREATMENT AND PREVENTION OF PROSTATIC HYPERPLASIA AND PROSTATITIS USING ROYSTONEA REGIA (ROYAL PALM) FRUITS**
PHARMAZEUTISCHE ZUSAMMENSETZUNG UND VERFAHREN ZUR BEHANDLUNG UND PRÄVENTION VON PROSTATAHYPERPLASIE UND PROSTATITIS UNTER VERWENDUNG VON FRÜCHTEN VON ROYSTONEA REGIA (KÖNIGSPALME)
COMPOSITION PHARMACEUTIQUE ET METHODE DE TRAITEMENT ET DE PREVENTION DE L'HYERPLASIE PROSTATIQUE ET DE LA PROSTATITE UTILISANT LES FRUITS DE ROYSTONEA REGIA (PALMIER ROYAL)

(30) Priority: 20.03.2003 CU 6203
(43) Date of publication of application: 08.02.2006
(73) Proprietor: Laboratorios Dalmer S.A., 12 100 Habana (CU)
(72) Inventor: LAGUNA GRANJA, Abilio M., 2da No.11007, 13 400 Habana 13 400 Habana (CU); RODRIGUEZ LEYES, Eduardo A., Dolores No. 274, 13 800 Habana (CU); MAS FERREIRO, Rosa M., Calle 206 No. 2113, 12 100 Habana (CU); CARBAJAL QUINTANA, Daisy, Calle 208 No. 1935, 12 100 Habana (CU); ARRUZAZABALA VALMANA, M.de Lourdes, Calle H No. 15010, 10 800 Habana (CU); MOLINA CUEVAS, Vivián, Calle 170 Edif. C-2 Apto 25, 12 100 Habana (CU); GONZALEZ CANAVACIOLO, Victor L., C. 174 Edif.DBE, 12 100 Habana (CU)
(74) Representative: Carpintero Lopez, Francisco
(86) International application number: PCT/CU2004/000004
(87) International publication number: WO 2004/082696

(56) References cited:
- CU-A1- 21 519
- EL-KHALAFY H.M. ET AL.: 'Chemical investigation of egyptian vegetable fats and oils. The chemical constitution of some anacardiaceae and palmae seed oils' GRASAS Y ACEITES vol. 22, no. 4, 1971, SEVILLA, ESPANA, pages 269 - 274, XP008060417
- DATABASE HCAPLUS [Online] 22 April 2004 BADAMI R.C. ET AL.: 'Fatty acids of Roystonea regia kernel and shell oils', XP002990399 Retrieved from STN Database accession no. 1980:474306 & JOURNAL OF THE OIL TEHCNOLOGISTS' ASSOCIATION OF INDIA vol. 11, no. 4, 1979, MUMBAI, INDIA, page 100
- DATABASE WPI Week 200258, Derwent Publications Ltd., London, GB; AN 2002-539219, XP002992863 & CN 1 184 670 A 17 June 1998

## Description

### Technical Section

This invention is related with the Pharmaceutical Industry, in particular with a new pharmaceutical composition and its obtention procedure from the Royal Palm (*Roystonea regia*) fruits, to prevent and/or treat benign prostatic hyperplasia (BPH) and prostatitis. The inventive composition consists in a mixture of free fatty acids and/or the esters thereof. Both fatty acids and/or the esters are within a range between 8 and 28 carbon atoms, especially between 8 and 18 carbon atoms. More especially, the mixture consists on saturated straight-chain fatty acids with 8, 10, 12, 14, 16 and 18 carbon atoms and monounsaturated fatty acids with 16:1 and 18:1 carbon atoms. The free fatty acids are enriched from the esters hydrolysis.

This composition is obtained from the Royal palm fruits, which are initially dried and grounded, this vegetable material submitted to a moderate basic hydrolysis up to obtain a saponified product, submitted to a selective extraction with organic solvents, or to a selective extraction of the mixture of fatty acids. Both procedures lead to compositions with pharmacological properties similar to the extract obtained without previous saponification.

The mixtures thus obtained are similar to the active ingredient contained in pharmaceutical different formulations for the treatment of BPH and prostatitis, as well as of other diseases, such as alopecia and hirsutism.

The present invention is related with the Pharmaceutical Industry, since the obtained pharmaceutical composition can be used as well, or in pharmaceutical formulations, as drugs used against BPH, prostatitis, alopecia and hirsutism.

### Previous Technique

The prostate is a gland located immediately below the bladder, and BPH is a disease present in more than 50% of the men older than 50 years old. BPH consists in the enlargement of the muscular fibre and the epithelial structure of the gland, that may cause an urinary obstruction that frequently requires surgery to improve the symptoms of the urinary retention, such as urinary disturbances, nyicturia included (Madsen and Bruskewitz, Curr Opinion Nephrol Hipert 4: 455-459, 1995). Meanwhile the surgery, particularly the transurethral resection, has been the mean treatment for the patients suffering this pathology, recently many other options have emerged (Oesterling, New Engl. J. Med 332: 99-109, 1995; Geller et al, J. Clin. Endocrinol Metab. 80, 745-756, 1995), which includes non-invasive surgical procedures and different drug treatments, like the inhibitors of the 5α-reductase enzyme (Boyle et al. Urology; 48:398-405, 1996), antagonists of the alpha-adrenergic receptors and phytotherapy extracts.

Male sexual hormones, mainly testosterone, play a very important role in the pathological etiology of this disease, being transformed via 5α-reductase in its more active derivative, the dihydrotestosterone (DHT), which binds to androgenic receptors, thus promoting the protein synthesis and consequent cellular growth. Hence, an increased activity of 5α-reductase could lead to BPH, since patients suffering of such pathology show DHT levels increased in 4 to 6 fold.

The exact cause of BPH is unknown, but clearly depends from the presence of the testes and its frequency increases with age. The androgen involved in prostate growth is DHT, which is formed in the prostate from testosterone, reaction catalyzed through the enzyme 5α-reductase. Conducted studies have demonstrated the prostate growth is directly related with the increase of DHT levels. On the other hand, in the man older than 50 years, estradiol production increases compared with androgens, being found that estrogens act synergistically with DHT to induce prostate hyperplasia in experimental models.

The symptoms of BPH are associated to the obstruction or irritation of the urethra, being disturbing for the patient. Among these symptoms, the following are included: nocturia, dysuria, reduced volume and strength of urinary flow, sensation of incomplete emptiness, and urinary retention. Most of the symptoms start gradually, but when the disorder progresses, the symptoms are worsened, being necessary to administer a pharmacological therapy.

Thus, α-adrenergic antagonists, like alfuzosin, doxazosin, terazosin, etc (Chapple, Eur. Urol 29, 129-144, 1996) and several plant extracts have been widely used to treat BPH. Currently, phytotherapy products are common in Europe and the United States, representing the 80 % of all drugs prescribed for the treatment of BPH.

In particular, extracts from the fruits of Saw palmetto (*Sabal serrulata,* syn. *Serenoa repens*) and from the roots of *Urtica dioica* are popular. Double-blinded, controlled clinical trials have been conducted to compare the effects of Saw palmetto extracts and finasteride, a potent inhibitor of the 5α-reductase, showing a similar efficacy between both treatments after 6 months on therapy (Carraro et al, Prostate 29, 231-240, 1996; Marks et al Journal of Urology 163: 1451-1456, 2000). On the other hand, an extract from the roots of *Urtica dioica* has been clinically used to treat BPH. Some histological and biological data demonstrated the effect of root extracts on the growth of androgen-dependent prostate cells.

The mechanism of action of Saw palmetto is not totally elucidated, and appears to include more than a single mechanism. Thus, investigations have proven that it can exert a non-competitive inhibition of alpha adrenergic receptors, suggesting a mechanism similar to tamsulosin. Other authors consider that Saw palmetto allows the epithelial contraction, possibly due to gland rupture, suggesting a mechanism similar to finasteride, involving the inhibition of the 5α reductase enzyme, which appears to be a fundamental mechanism (Niederprum et al, Annals N.Y. Acad Sci, 768, 227-30, 1995).

The androgenic alopecia is common in both men and women, being androgen-dependent, since hairless is directly related with the levels of the 5 α- reductase enzyme, the hairless-related androgen being DHT, produced from testosterone through the action of this enzyme. Thus, increased levels of 5 α- reductase enzyme have been detected in the frontal zone of hairless men (Bingham KD et al, J. Endocrinology 57, 111, 1973), while men with the syndrome of deficiency of 5 α- reductase enzyme do not suffer hair loss (Ebling FJ, Clin Endocrinol Metab 319, 1986).

Hair loss has been demonstrated as a widely distributed feature that can start as soon as after puberty, that is why is necessary to develop products to prevent hairless in both men and women.

As known, the lipid extracts from *Saw palmetto* inhibit the production of the DHT hormone, blocking the 50 % of the binding sites of DHT to the receptors and its entry to the nuclei of the prostate cells, strongly inhibiting the activity of 5 α- reductase enzyme. Therefore, the extracts of the fruits of such species have been used in the treatment of alopecia (WO9702041, USA 6,019,976 y WO9833472).

The extract from *Saw palmetto* commercially used to treat BPH is a mixture of fatty acids, containing sterols and high molecular weight alcohols, obtained from plant fruits as per different reported methods (EPO541853, EPO492305, EPO250953, USA Patent 6,039,950, Cristoni, Fitoterapia 68, 355, 1997; DeSwaef Nat Prod Letters 7, 223, 1996). Few authors, however, have reported a method so simple and economic like that claimed in the present invention for the attainment of the fruits of an *Arecaceae*, in the present case, the fruits of *Roystonea regia.*

### Divulgation of the Invention

The procedure for the obtention of the composition object of the current inventions is based on the drying of the mature fruits of *Roystonea regia.* Dry fruits are grounded up to obtain a fine powder, with a particle size < 5000 µm. This powder is later submitted or not to basic hydrolysis performed with alkaline, alkaline-earthen and organic hydroxides, especially with low molecular weight hydroxides, and more especially with sodium, potassium, calcium or ammonium hydroxides; followed by a selective extraction in organic solvents or supercritical with CO₂.

The vegetal material hydrolysed or not is extracted in a conventional solid liquid extractor , wherein that fatty acids and/or the esters are separated from other components present in fruits though a selective extraction in the adequate solvent, like alcohols between 1 to 3 carbon atoms and hydrocarbons between 5 to 8 carbon atoms. Among such solvents, methanol, ethanol, 2-propanol, hexane, pentane, isopentane, heptane and octane are included.

The obtained yield is between 5 and 20 %. The composition of the mixture of acids obtained includes saturated acids of 12, 14, 16 and 18 carbon atoms, as well as the unsaturated with 16:1 and 18:1 carbon atoms, whose qualitative and quantitative composition is reported in Table 1.

**Table 1. Composition of the fatty acids present in the lipid extract of Roystonea regia**

| **Component of acids** | **Relative percent in the mixture** |
|---|---|
| Caprylic acid (C8:0) | < 3.0 |
| Capric acid (C10:0) | < 3.0 |
| Lauric acid (C12:0) | 3.0 - 40.0 |
| Miristic acid (C14:0) | 4.0 -15.0 |
| Palmitic acid (C 16:0) | 10.0 - 80.0 |
| Palmitoleic acid (C16:1) | 0.15 - 20.0 |
| Estearic acid (C18:0) | 0.1 - 5.0 |
| Oleic acid (Cl 8: 1) | 3.0 - 50.0 * |

| | |
|---|---|
| ** Quantified* as *the total of the chromatographic peak in which the oleic acid elutes (60 - 70 % of the peak), linoleic acid* (*25 - 40 % of the peak) and linolenic acid* (*< 10 % of the chromatographic peak)* | |

An overall assessment of the substance obtained from *Roystonea regia* fruits as per the current invention shows that such composition is very safe and well tolerated, a relevant advantage supported through the results of the toxicity tests conducted in rodents, which revealed no substance-related toxicity.

The aim of the current invention will be described in detail, being referred to, but not limited to, the following examples.

### Example 1

Fresh fruits 5 kg of *Roystonea regia* are taken, and placed in an oven at controlled temperature 45 °C for 7 days. In a grinding mill, dry fruits are ground to a particle size between 1500 and 2000 µm, and 1000 g from such powder are taken and placed in an agitating reactor, being submitted to alkaline hydrolysis. Later on, the product is extracted with 10 L of hexane, heating up to 55 °C and constantly stirring for 36 hours. Then, the product is filtered and evaporated to dryness at 50 °C, using vacuum. The weight of the obtained extract is 98,5 g, and table 2 shows its composition determined throughout gas chromatography.

**Table2. Composition of the fatty acids present in the lipid extract of Roystonea regia**

| **Component of acids** | **Relative percent in the mixture** |
|---|---|
| Caprylic acid (C8:0) | 1.0 |
| Capric acid (C10:0) | 1.0 |
| Lauric acid (C12:0) | 5.7 |
| Miristic acid (C14:0) | 4.4 |
| Palmitic acid (C16:0) | 75.1 |
| Palmitoleic acid (C16:1) | 1.9 |
| Estearic acid (C18:0) | 1.9 |
| Oleic acid (C18:1) | 9.1 |

### Example 2

Fresh fruits 10 kg of *Roystonea regia* are placed in an oven at controlled temperature 45 °C for 7 days. In a grinding mill, dry fruits are ground to a particle size < 1500 µm, and 1500 g from such powder are taken and submitted to alkaline hydrolysis. Later on, the product is extracted at 60 °C for 48 hours in a Söxhlet extractor containing 10 L of ethanol. Then, the organic solution is removed, filtered and evaporated to dryness at 50 °C, using vacuum. The obtained extract is weighed and analysed through gas chromatography, its composition being shown in Table 3.

**Table 3. Composition of the fatty acids present in the lipid extract of Roystonea regia**

| **Component of acids** | **Relative percent in the mixture** |
|---|---|
| Caprylic acid (C8:0) | 1.0 |
| Capric acid (C10:0) | 1.0 |
| Lauric acid (C12:0) | 3.2 |
| Miristic acid (C14:0) | 5.5 |
| Palmitic acid (C16:0) | 27.1 |
| Palmitoleic acid (C16:1) | 18.5 |
| Estearic acid (C18:0) | 0.3 |
| Oleic acid (C18:1) | 43.4 |

### Example 3

Fresh fruits 5 kg of *Roystonea regia* are taken, and placed in an oven at controlled temperature 45 °C for 7 days. In a grinding mill, dry fruits are ground to a particle size between 1500 and 1800 µm, and 1000 g from such powder are taken and submitted to alkaline hydrolysis. Later on, the product is extracted in an agitating reactor containing 10 L of heptane at 60 °C, with constant stirring, for 50 hours. Then, the solvent is removed and evaporated to dryness at 65 °C, using vacuum. The obtained extract is weighed and analysed through gas chromatography, showing the composition summarised in the Table 4.

**Table 4. Composition of the fatty acids present in the lipid extract of Roystonea regia**

| **Component of acids** | **Relative percent in the mixture** |
|---|---|
| Caprylic acid (C8:0) | 1.0 |
| Capric acid (C10:0) | 1.0 |
| Lauric acid (C12:0) | 3.16 |
| Miristic acid (C14:0) | 12.7 |
| Palmitic acid (C16:0) | 13.0 |
| Palmitoleic acid (C 16: 1) | 1.7 |
| Estearic acid (C 18:0) | 2.3 |
| Oleic acid (C18:1) | 36.6 |

### Example 4

Fresh fruits 5 kg of *Roystonea regia* are taken, and placed in an oven at controlled temperature 45 °C for 7 days. In a grinding mill, dry fruits are ground to a particle size between 1000 and 1800 µm, and 1000 g from such powder are taken and treated with ammonium hydroxide up to moisten all the power. Later on, the powder is extracted in an agitating reactor containing 10 L of hexane at 55 °C, with constant stirring, for 36 hours. Then, the solvent is removed and evaporated to dryness at 50 °C, using vacuum. The obtained extract is weighed and analysed through gas chromatography, showing the composition summarised in the Table 5.

**Table 5. Composition of the fatty acids present in the lipid extract of Roystonea regia**

| **Component of acids** | **Relative percent in the mixture** |
|---|---|
| Caprylic acid (C8:0) | 1.0 |
| Capric acid (C10:0) | 1.0 |
| Lauric acid (C12:0) | 19.7 |
| Miristic acid (C14:0) | 9.7 |
| Palmitic acid (C16:0) | 14.8 |
| Palmitoleic acid (C16:1) | 0.2 |
| Estearic acid (C 18:0) | 3.8 |
| Oleic acid (C18:1) | 49.8 |

### Example 5

To assess the effect of the lipid extract from *Roystonea regia* fruits, OF1 male albine mice weighing between 30 and 40 g were adapted for 7 days to laboratory conditions, with controlled temperature (25 ± 2°C) and humidity. After the adaptation period, the animals were randomly distributed in different experimental groups. The extract from *Roystonea regia* was suspended in a Tween-20/H₂O vehicle and treatments were administered orally (5mL/kg.), control animal being treated with the vehicle. The deposit testosterone was dissolved in vegetal oil and injected by intramuscular (i.m.) route at 100 mg/kg, as a single dosing. Animals were randomly distributed in the following groups: 1) Negative control 2) positive control (animals injected with testosterone and orally administered with the vehicle) 3, 4 and 5) animals injected by intramuscular (im) route with testosterone and orally treated with the extract of *Roystonea regia* fruits at 200, 400 and 800 mg/kg, respectively and 6) animals injected with testosterone and orally administered with the extract of *Saw palmetto* fruits at 400 mg/kg.

Once concluded the dosing period (14 days) mice were sacrificed, their abdomen opened through an incision in the ventral mean line, the prostates separated and weighed. The comparison between treated and control groups were done through the Mann Whitney U test. Table 6 shows the results.

As observed, deposit testosterone (100 mg/kg) im injected produced a significant increase of prostate weight and prostate weight to bodyweight ratio compared with the negative control group.

**Table 6 Effect of the lipid extract form Roystonea regia fruits in mice with induced prostate hyperlasia (PH)**

| **Treatment.** | **Doses (mg/kg)** | **n** | **BW** | **PW** | **%I(PW)** | **PW/BW** | **%I** (PW/BW) |
|---|---|---|---|---|---|---|---|
| **Control -** | | 10 | 36.0 ± 0.7 | 25.7 ± 3.2 | | 0.72 ± 0.09 | - |
| **Control +** | | 10 | 35.5 ± 0.68 | 54.7 ± 3.8+++ | | 1.55 ± 0.12+++ | - |
| ***Roystonea regia*** | 200 | 10 | 35.4 ± 0.77 | 38.7 ± 2.6 ** | 55.1 | 1.09 ± 0.08 * | 55.4 |
| ***Roystonea regia*** | 400 | 9 | 33.7 ± 1.2 | 32.3 ± 1.2 ** | 77.2 | 0.96 ± 0.03 ** | 71.8 |
| ***Roystonea regia*** | 800 | 11 | 33.6 ± 0.7 | 26.6 ± 1.9 *** | 96.6 | 0.78 ± 0.04 *** | 92.7 |
| ***Saw palmetto*** | 400 | 8 | 34.3 ± 0.84 | 36.6 ± 2.5 ** | 62.1 | 1.06 ± 0.06 ** | 59.0 |

| | | | | | | | |
|---|---|---|---|---|---|---|---|
| BW bodyweight (g), PW prostate weight (mg), % I percent inhibition, +++ p<0.001 Sham vs control, Mann Whitney U test, *p< 0-05, ** p< 0.01, ***p<0.001 comparison vs control. | | | | | | | |

The oral treatment with the inventive composition (200-800 mg/kg.) for 2 weeks inhibited significantly and dose-dependently the increase of prostate size induced with testosterone achieving a percent inhibition of 96.6% with the highest dose (800 mg/kg), which also inhibited significantly and dose-dependently the increase the ratio of prostate weight/bodyweight, reaching a percent inhibition of 92.7% with 800 mg/kg. On the other hand, treatment with repeated doses of *Saw palmetto* (400 mg/kg) inhibited prostate enlargement in 62 % and reduced the ratio of PW/BW by 59%, resulting less effective than the extract of *Roystonea regia* fruits, which at a similar dose produced an inhibition of 77 and 72 % respectively.

The results obtained in this experiment demonstrate the efficacy of the lipid extract of *Roystonea regia* fruits in PH in mice, showing a dose-dependent reduction of prostate size, reaching a percent inhibition of 77.2%.
at 400 mg/kg achieving a percent inhibition greater than that induced with a similar dose of *Saw palmetto.*

### Example 6

To assess the effect of the lipid extract from *Roystonea regia* fruits, Sprague Dawley male rats weighing between 250 and 270 g were adapted for 7 days to laboratory conditions, with controlled temperature (25 ± 2°C) and humidity. After the adaptation period, the animals were randomly distributed in different experimental groups.

The extract from *Roystonea regia* fruits was suspended in a Tween-20/H₂O vehicle and treatments were administered orally (5mL/kg.), control animal being administered with the vehicle. Testosterone propionate was dissolved in vegetal oil and injected through subcutaneous sc route at 4 mg/kg for 14 days.

The rats were randomly distributed in 6 experimental groups: 1) Negative control + (vegetal oil), 2) Positive Control (vehicle) + testosterone; 3, 4 and 5) extract from the fruits of *Roystonea regia* at 50, 200 and 400 mg/kg. Once concluded the dosing period (2 weeks) rats were sacrificed and bleed to excess, the abdomen opened through an incision in the ventral mean line, the prostates separated and weighed. The comparison between treated and control groups were done through the Mann Whitney U test. Table 7 shows the results.

**Table 7. Effect of the lipid extract from Roystonea regia fruits on rats with induced prostate hyperplasia (PH)**

| **Treatment** | **Doses (mg/kg)** | **n** | **BW** | **PW** | **% I (PW)** | **PW/BW** | **% I(PW/ PB)** |
|---|---|---|---|---|---|---|---|
| **Control -** | 0 | 10 | 354.7 ± 6.9 | 576.03 ± 51.4 | - | 1.6 ± 0.15 | |
| **Control +** | 0 | 10 | 339.5 ± 6.5 | 916.07 ± 34.4 +++ | - | 2.7 ± 0.12 +++ | - |
| *R regia* | 50 | 9 | 322.5 ± 10.2 | 895.4 ± 34.6 | 6 | 2.7 ± 0.11 | 0 |
| *R regia* | 200 | 9 | 335.3 ± 6.4 | 838.05 ± 30.9 | 23 | 2.5 ± 0.09 | 19 |
| *R regia* | 400 | 10 | 342.4 ± 6.4 | 776.01 ± 35.4* | 41 | 2.2 ± 0.11 * | 45 |

| | | | | | | | |
|---|---|---|---|---|---|---|---|
| BW bodyweight (g), PW prostate weight (mg), % I percent inhibition, +++ p<0.0001 Sham vs control, * p< 0.05, ** p< 0.01, *** comparison vs control, Mann Whitney U test | | | | | | | |

As observed, subcutaneous injection with testosterone 4 mg/kg for 14 days significantly increased the prostate weight and the ratio of prostate weight/bodyweight, compared with the negative control. The oral dosing with the extracts of *Roystonea regia* and *Saw palmetto* (400 mg/kg) fruits for 2 weeks significantly inhibited the increase of prostate size induced with testosterone injection, achieving a percent inhibition of 41 and 58 % respectively. At 400 mg/kg, the extract from *Roystonea regia* fruits also decreased significantly the prostate weight/bodyweight ratio, achieving an inhibition of 45 %.

The results obtained in this study indicate the efficacy of the treatment with the extract of *Roystonea regia* fruits at 400 mg/kg for 14 days to rats treated with testosterone, producing an inhibition of the prostate size increase respect to bodyweight similar for both treatments (45%), favourable to the potential therapeutic use of extract of the royal palm fruits.

### Example 7

To assess the effect of the extract of *Roystonea regia* fruits, Sprague Dawley male rats weighing between 250 and 270 g were adapted for 7 days to laboratory conditions, with controlled temperature (25 ± 2°C) and humidity. After the adaptation period, the animals were randomly distributed in different experimental groups. Testosterone propionate was dissolved in vegetal oil and administered through subcutaneous route at daily doses of 3 and 4 mg/kg for 14 days.

The extract of *Roystonea regia* fruits was suspended in a Tween-20/H₂O vehicle and treatments were administered orally (5mL/kg.), control animals being administered with the vehicle.

The rats treated with testosterone 3mg/kg were distributed in 5 experimental groups: 1) Negative control + (vegetal oil), 2) positive control (vehicle) + testosterone; 3,4) *Roystonea regia* extracts at 50 and 200 mg/kg + testosterone; 5) Saw palmetto 200 mg/kg + testosterone. In turn, animals treated with testosterone 4mg/kg were distributed in 6 experimental group: 1) Negative control + (vegetal oil), 2) positive control (vehicle) + testosterone; 3, 4, 5) Roystonea regia extracts at 50, 200 and 400 mg/kg + testosterone; 6) Saw palmetto 400 mg/kg + testosterone. All oral treatments were administered for 14 days. Tables 8 and 9 show the results.

**Table 8. Effect of the lipid extract from Roystonea regia fruits on rats with induced prostate hyperplasia (PH) Doses Treatment**

| **Treatment** | **Doses (mg/kg)** | **n** | **BW** | **PW** |
|---|---|---|---|---|
| **Control -** | 0 | 9 | 507.0 ± 35 | 1.43 ± 0.09 |
| **Control +** | 0 | 8 | 886.0 ± 78.8 ++ | 2.69 ± 0.26 +++ |
| **Roystone a regia** | 50 | 10 | 684.8 ± 34.7 * | 2.05 ± 0.11 * |
| ***Roystonea regia*** | 200 | 10 | 693.1 ± 36.5* | 2.11 ± 0.13 ^{a} |
| ***Saw Palmetto*** | 200 | 10 | 710.0 ± 43.3 t | 2.08 ± 0.14 ^{a} |

| | | | | |
|---|---|---|---|---|
| BW bodyweight (g), PW prostate weight (mg), ++ p<0.01; +++ p<0.001 comparison vs Sham; *p<0.05; ^{a}: p=0.05 comparison vs control (Mann Whitney U test) | | | | |

**Table 9. Effect of the lipid extract from Roystonea regia fruits on rats with induced prostate hyperplasia (PH)**

| **Treatment** | **Doses (mg/kg)** | **n** | **BW** | **PW** | **BW/PW** | **% I** |
|---|---|---|---|---|---|---|
| **Control** - | 0 | 10 | 354.7 ± 6.90 | 576.03 ± 51.4 | 1.6 ± 0.15 5 | - |
| ***Control +*** | 0 | 10 | 339.5 ± 6.50 | 916.07 ± 34.4++++ | 2.7 ± 0.12 +++ | - |
| ***Roystonea regia*** | 50 | 9 | 322.5 ± 10.2 | 895.40 ± 34.6 | 2.7 ± 0.11 | 6.0 |
| ***Roystonea regia*** | 200 | 9 | 335.3 ± 6.40 | 838.05 ± 30.9 | 2.5 ± 0.09 | 22.9 |
| ***Roystonea regia*** | 400 | 10 | 342.4 ± 6.40 | 776.01 ± 35.4 * | 2.2 ± 0.11 * | 41.2 |
| ***Saw palmetto*** | 400 | 10 | 328.5 ± 6.80 | 720.1 ± 41.8 ** * | 2.2 ± 0.12 * | 57.6 |

| | | | | | | |
|---|---|---|---|---|---|---|
| BW bodyweight (g), PW prostate weight (mg), % I percent inhibition, ++ p< 0.01; +++ p< 0.001 comparison vs Sham;* p<0.05; p<0.01, t: p=0.06 comparison vs control (Mann Whitney U test) | | | | | | |

### Example 8

Rabbits with a bodyweight between 4 and 5 kg were used in the study. At baseline and after treatment with the extract of *Roystonea regia* fruits, the hair growth of each rabbit was measured. Hair measurement was done before, during and after the oral treatment with the extract of *Roystonea regia* fruits, with a 4 week interval between every measurement. After sedation of each animal, a dorsal area of approximately one square inch was shaved and the hair of such area was weighed.

Shaved rabbits were distributed in 4 groups of 5 rabbits each, the rabbits of the group 1 received once a day a daily administration of the extract of *Roystonea regia* 400 mg, while rabbits of the group 2 received once a day a daily administration of vehicle. In turn, the rabbits of the group 3 received a daily administration of the extract of *Roystonea regia* 400 mg distributed in two doses of 200 mg, and rabbits of the group 4 received vehicle distributed in two dosing, like group 3.

The changes in the weight of the hair growing from the indicated areas occurred were compared using a Variance Analysis for between group comparisons and the Wilcoxon test for paired simples for within group changes. The changes were considered as significantly different for p <0, 05.

The results of this study demonstrate that the hair growth of the rabbits from groups 1 and 3 was significantly superior than hair growth of the rabbits from groups 2 and 4, and also superior to the initial hair weight, demonstrating that extract from *Roystonea regia* could be used to treat alopecia.

## Claims

1. Pharmaceutical composition obtained from the green or mature fruits of *Roystonea regia,* which contains a mixture of the primary fatty acids with 8 to 28 carbon atoms, including the following fatty acids: caprilic (C8:0), capric (C10:0), lauric (C12:0), miristic (C14:0), palmitic (C16:0), palmitoleic (C16:1), estearic (C18:0) and oleic (C18:1) (which includes oleic acid itself, linoleic and linolenic acids), as well as a mixture of the esters of such fatty acids. The free fatty acids are enriched from the esters hydrolisis.

2. Pharmaceutical composition which contains a mixture of the primary fatty acids and the esters thereof, according to the claim 1, **characterized by** the following composition in fatty acids
Mixture of fatty acids present in the lipid extract of *Roystonea regia* fruits
Caprylic acid (C8:0) < 3.0 %
Capric acid (C10:0) < 3.0 %
Lauric acid (C12:0) 3.0 - 40.0 %
Miristic acid (C14:0) 4.0 - 15.0 %
Palmitic acid (C16:0) 10.0 - 80.0 %
Palmitoleic acid (C16:1) 1.5 - 20.0 %
Estearic acid (C18:0) 0.1 - 5.0 %
Oleic acid (C18:1) 3.0 - 50.0 %

3. Method for the obtention of the pharmaceutical composition obtained from *Roystonea regia* according to the claim 1, and 2 including the drying, ground and sieving of *Roystonea regia* fruits, and a further separation of the extract from other components through a solid/liquid extraction in organic solvents like hydrocarbons of 5 to 8 carbon atoms, alcohols of 1 to 3 carbon atoms, as well as mixture of them, with or without a previous basic hydrolysis using hydroxides or alkalis.

4. Method of obtention according to the claim 3 , including the drying of *Roystonea regia* fruits at a temperature between 10 and 100 °C for a time ranging from 1 to 1000 hours, and further ground in a proper mill, that allow obtain a particle size < 6000 µm. The time for the extraction of the components of the active extract ranges from 1 to 50 h and the temperature from 0 to 70 °C.

5. Method of obtention according to the claim 3 and 4, **characterized by** the use of alkaline- hydroxides or alkaline-earthen hydroxides and organic for the basic hydrolisis, especifically those of low molecular weight and more specifically sodium, potassium, calcium or ammonium hydroxides.

6. Method of obtention according to the claim 3 and 4, **characterized by** the use of hydrocarbons like pentane, hexane, heptane or octane for the obtention of the extract containing the inventive mixture of fatty acids.

7. Method of obtention according to the claim 3 and 4, **characterized by** the use of alcohols like methanol, ethanol, n-propanol y 2-propanol for the obtention of the extract containing the inventive mixture of fatty acids.

8. Pharmaceutical composition as per the claims 1 and 2 **characterized by** its use as medication in the treatment and/or to prevent of BPH, prostatitis, alopecia and hirsutism.

9. Pharmaceutical composition with or without a saponification, as per the claims 1 and 2, **characterized by** its use as medication in the treatment and/or to prevent BPH, prostatitis, alopecia and hirsutism.

10. Pharmaceutical composition with or without a saponification, as per the claims 1, 2, 8 and 9, **characterized by** its use, at daily doses from 50 to 1000 mg, specifically formulated at doses between 150 and 1000 mg, to be administered as solid oral forms (capsules, soft-gel capsules, tablets) or liquids (emulsions), as supossitories or like tinctures, lotions or shampoos of local action in the treatment and/or prevention of BPH, prostatitis, alopecia and hirsutism.

## Patentansprüche

1. Pharmazeutische Zusammensetzung, die aus den grünen oder reifen Früchten von *Roystonea regia* erhalten wird, welche eine Mischung aus den primären Fettsauren mit 8 bis 28 Kohlenstoffatomen, einschließlich der folgenden Fettsäuren, enthält: Caprylsäure (C8:0), Caprinsäure (C10:0), Laurinsäure (C12:0) , Myristinsäure (C14:0) , Palmitinsäure (C16:0), Palmitoleinsäure (C16:1), Stearinsäure (C18:0) und Ölsäure (C18:1) (welche Ölsäure selbst, Linol- und Linolensäure einschließt) sowie eine Mischung der Ester von solchen Fettsäuren. Die freien Fettsäuren werden aus der Esterhydrolyse angereichert.

2. Pharmazeutische Zusammensetzung, welche eine Mischung von primären Fettsäuren und den Estern davon, gemäß Anspruch 1, enthält, **gekennzeichnet durch** die folgende Zusammensetzung in Fettsäuren: Mischung von Fettsäuren, die in dem Lipidextrakt von *Roystonea regia*-Früchten vorliegen
Caprylsäure (C8:0) < 3,0 %
Caprinsäure (C10:0) < 3,0 %
Laurinsäure (C12:0) 3,0-40,0 %
Myristinsäure (C14:0) 4,0-15,0 %
Palmitinsäure (C16:0) 10,0-80,0 %
Palmitoleinsäure (C16:1) 1,5-20,0 %
Stearinsäure (C18:0) 0,1-5,0 %
Ölsäure (C18:1) 3,0-50,0 %

3. Verfahren zum Erhalt der pharmazeutischen Zusammensetzung die aus *Roystonea regia* gemäß Anspruch 1 und 2 erhalten wird, einschließlich dem Trocknen, Mahlen und Sieben von *Roystonea* regia-Früchten, und einer weiteren Abtrennung des Extraktes von anderen Komponenten durch eine Fest/Flüssig-Extraktion in organischen Lösungsmitteln wie Kohlenwasserstoffen mit 5 bis 8 Kohlenstoffatomen, Alkoholen mit 1 bis 3 Kohlenstoffatomen, sowie einer Mischung von diesen mit oder ohne einer vorausgehenden basischen Hydrolyse unter Verwendung von Hydroxiden oder Alkalien.

4. Verfahren zum Erhalt gemäß dem Anspruch 3, einschließlich dem Trocknen von *Roystonea regia-*Früchten bei einer Temperatur zwischen 10 und 100 °C für eine Zeit im Bereich von 1 bis 1000 Stunden, und ferner dem Mahlen in einer angemessenen Mühle, welche es ermöglicht, eine Teilchengröße < 6000 µm zu erhalten. Die Zeit zur Extraktion der Komponenten des aktiven Extraktes liegt im Bereich von 1 bis 50 h und einer Temperatur von 0 bis 70 °C. oder

5. Verfahren zum Erhalt gemäß Anspruch 3 und 4, **gekennzeichnet durch** die Verwendung von Alkalihydroxiden und Erdalkalihydroxiden und organischen Stoffen für die basische Hydrolyse, insbesondere jenen mit niedrigem Molekulargewicht und spezifischer Natrium-, Kalium-, Calcium- oder Ammoniumhydroxiden

6. Verfahren zum Erhalt gemäß dem Anspruch 3 und 4, **gekennzeichnet durch** die Verwendung von Kohlenwasserstoffen wie Pentan, Hexan, Heptan oder Oktan zum Erhalt des Extraktes, der die erfindungsgemäße Mischung von Fettsäuren enthält.

7. Verfahren zum Erhalt gemäß dem Anspruch 3 und 4, **gekennzeichnet durch** die Verwendung von Alkoholen wie Methanol, Ethanol, n-Propanol und 2 -Propanol zum Erhalt des Extraktes, welcher die erfindungsgemäße Mischung von Fettsäuren enthält.

8. Pharmazeutische Zusammensetzung gemäß Anspruch 1 und 2, **gekennzeichnet durch** dessen Verwendung als Medikament bei der Behandlung und/oder zur Verhinderung von BPH, Prostatitis, Alopecia und Hirsutismus.

9. Pharmazeutische Zusammensetzung mit oder ohne Verseifung gemäß Anspruch 1 und 2, **gekennzeichnet durch** ihre Verwendung als Medikament bei der Behandlung und/oder Verhinderung von BPH, Prostatitis, Alopecia und Hirsutismus.

10. Pharmazeutische Zusammensetzung mit oder ohne Verseifung gemäß Anspruch 1, 2, 8 und 9, **gekennzeichnet durch** ihre Verwendung als tägliche Dosis von 50 bis 1000 mg, insbesondere formuliert zu Dosen zwischen 150 und 1000 mg, die als feste orale Formen (Kapseln, Weichgelkapseln, Tabletten) oder Flüssigkeiten (Emulsionen) , als Zäpfchen oder ähnlichen Tinkturen, Lotionen oder Shampoos zur lokalen Wirkung bei der Behandlung und/oder Verhinderung von BPH, Prostatitis, Alopecia und Hirsutismus zu verabreichen sind.

## Revendications

1. Composition pharmaceutique obtenue à partir des fruits verts ou mûrs de *Roystonea regia,* qui contient un mélange d'acides gras primaires avec 8 à 28 atomes de carbone, comprenant les acides gras suivants : acide caprylique (C8:0), acide caprique (C10:0), acide laurique (C12:0), acide myristique (C14:0), acide palmitique (C16:0), acide palmitoléique (C16:1), acide estéarique (C18:0) et acide oléique (C18:1) (qui comprend l'acide oléique même, les acides linoléique et linolénique), ainsi qu'un mélange des esters de tels acides gras, les acides gras libres étant enrichis par l'hydrolyse des esters.

2. Composition pharmaceutique contenant un mélange des acides gras primaires et des esters de ceux-ci, selon la revendication 1, **caractérisée par** la composition suivante en acides gras .
Mélange d'acides gras présents dans l'extrait de lipide des fruits de *Roystonea regia*
Acide caprylique (C8:0) < 3,0 %
Acide caprique (C10:0) < 3,0 %
Acide laurique (C12:0) 3,0 - 40,0 %
Acide myristique (C14:0) 4,0 - 15,0 %
Acide palmitique (C16:0) 10,0 - 80,0 %
Acide palmitoléique (C16:1) 1,5 - 20,0 %
Acide estéarique (C18:0) 0,1 - 5,0 %
Acide oléique (C18:1) 3,0 - 50,0 %

3. Procédé d'obtention de la composition pharmaceutique obtenue à partir de *Roystonea regia* selon les revendications 1 et 2, comprenant le fait de sécher, de moudre et de tamiser des fruits de *Roystonea regia,* et une autre séparation de l'extrait provenant d'autres composants au moyen d'une extraction de solide/liquide dans des solvants organiques tels que les hydrocarbures ayant de 5 à 8 atomes de carbone, les alcools ayant de 1 à 3 atomes de carbone, ainsi qu'un mélange de ceux-ci, avec ou sans une hydrolyse basique préalable qui utilise des hydroxydes ou des alcalis.

4. Procédé d'obtention selon la revendication 3, comprenant le fait de sécher de fruits de *Roystonea regia* à une température située entre 10 et 100 °C pendant une durée allant de 1 à 1000 heures, et en outre le fait de moudre dans un broyeur adapté, qui permet d'obtenir une taille de particules < 6000 µm, la durée de l'extraction des composants de l'extrait actif variant de 1 à 50 h et la température de 0 à 70 °C.

5. Procédé d'obtention selon les revendications 3 et 4, **caractérisé par** l'utilisation d'hydroxydes alcalins ou d'hydroxydes alcalino-terreux et organiques pour l'hydrolyse basique, en particulier ceux ayant une faible masse moléculaire et plus particulièrement les hydroxydes de sodium, de potassium, de calcium ou d'ammonium.

6. Procédé d'obtention selon les revendications 3 et 4, **caractérisé par** l'utilisation d'hydrocarbures tels que le pentane, l'hexane, l'heptane ou l'octane pour l'obtention de l'extrait contenant le mélange d'acides gras de l'invention.

7. Procédé d'obtention selon les revendications 3 et 4, **caractérisé par** l'utilisation d'alcools tels que le méthanol, l'éthanol, le n-propanol y 2-propanol pour l'obtention de l'extrait contenant le mélange d'acides gras de l'invention.

8. Composition pharmaceutique selon les revendications 1 et 2, **caractérisée par** son utilisation en tant que médicament dans le traitement et/ou en prévention de l'hyperplasie prostatique bénigne, de la prostatite, de l'alopécie et de l'hirsutisme.

9. Composition pharmaceutique avec ou sans saponification, selon les revendications 1 et 2, **caractérisée par** son utilisation en tant que médicament dans le traitement et/ou en prévention de l'hyperplasie prostatique bénigne, de la prostatite, de l'alopécie et de l'hirsutisme.

10. Composition pharmaceutique avec ou sans saponification, selon les revendications 1, 2, 8 et 9, **caractérisée par** son utilisation, à doses quotidiennes allant de 50 à 1000 mg, formulée spécifiquement à des doses entre 150 et 1000 mg, devant être administrée sous formes orales solides (capsules, capsules molles, comprimés) ou liquides (émulsions), de suppositoires ou de teintures, de lotions ou de shampooings ayant une action locale dans le traitement et/ou la prévention de l'hyperplasie prostatique bénigne, de la prostatite, de l'alopécie et de l'hirsutisme.
